# EUROPÄISCHE PATENTSCHRIFT

(11) **EP 1 318 774 B1**
(45) Veröffentlichungstag und Bekanntmachung des Hinweises auf die Patenterteilung: **25.05.2005**
(21) Anmeldenummer: 01967049.6
(22) Anmeldetag: 25.08.2001
(51) Int. Cl.: A61F 2/24

(54) **PROTHETISCHE MITRAL-HERZKLAPPE**
PROSTHETIC MITRAL HEART VALVE
VALVULE MITRALE PROTHETIQUE

(30) Priorität: 19.09.2000 DE 10046550
(43) Veröffentlichungstag der Anmeldung: 18.06.2003
(73) Patentinhaber: ADIAM life science AG, 41812 Erkelenz (DE)
(72) Erfinder: JANSEN, Josef, 50937 Köln (DE)
(74) Vertreter: Vomberg, Friedhelm, Dipl.-Phys.
(86) Internationale Anmeldenummer: PCT/DE2001/003333
(87) Internationale Veröffentlichungsnummer: WO 2002/024117

(56) Entgegenhaltungen:
- WO-A-97/49355
- US-A- 4 425 670
- US-A- 4 759 759

## Beschreibung

Die Erfindung betrifft eine prothetische Mitral-Herzklappe, gemäß dem Oberbegriff von Anspruch 1.

Eine solche Mitral-Herzklappe ist aus der WO 97/49355 bekannt. Um eine potentielle gegenseitige Funktionsbeeinträchtigung des Herzens und der Klappe zu vermeiden, wird in dieser Druckschrift vorgeschlagen, daß die Wandung mit geringerer Krümmung ein unter einem zur Basisringgrundfläche stärker geneigten Winkel angeordnetes flächenkleineres (murales) Segel trägt als die Wandung mit größerer Krümmung. Die beiden Halbformen des Basisrings bilden somit einen Stent-Körper, der eine D- oder Nieren-Form aufweist, die der natürlichen Mitral-Klappe des Herzens weitgehend angenähert sein soll. Um das Risiko der Interferenz und eine mögliche Beeinträchtigung des Stützgehäuses und der kontrahierenden Herzkammerinnenwand zu verringern, wird weiterhin vorgeschlagen, die Hauptströmungsrichtung um 10° bis 25°, vorzugsweise um 15° von der normalen zum muralen Segel zu neigen. Die Segel sollen einen ausgeprägten trichterförmigen Öffnungskanal mit einem im Vergleich zu einer Aortenklappe geringeren Öffnungsquerschnitt bilden. Obwohl diese Anordnung und Gestalt der Mitral-Herzklappe eine günstige physiologische Strömungsführung vom Vorhof in den Ventrikel gewährleistet und die Herzklappe in geringer Bauhöhe im Vergleich zu den bis zu diesem Zeitpunkt nach dem Stand der Technik bekannten Ausführungen mit einem kreisförmigen oder symmetrisch ellipsenförmigen Stützgehäusequerschnitt, lassen sich beim Durchströmen Turbulenzen nicht vollständig vermeiden, die in den Bereichen auftreten, in denen die Stentinnenwandung in Bezug auf die Durchströmrichtung Hinterschneidungen bildet, d.h. Bereiche, die hinter vorstehenden Kanten liegen und die bezogen auf den Durchstromfluß Nischen bilden, welche die Ausbildung von unerwünschten Strömungsturbulenzen fördern.

Es ist Aufgabe der vorliegenden Erfindung, die genannte Mitral-Herzklappe durch konstruktive Änderung dahingehend zu verbessern, daß solche turbulenzenfördernde Hinterschneidungen vermieden werden.

Diese Aufgabe wird durch die prothetische Mitral-Klappe nach Anspruch 1 gelöst. Dadurch wird ein Stützgehäuse geschaffen, dessen Strömungsrichtung durch entsprechende Neigung der Wandungen gegenüber der Basisringachsrichtung um einen Winkel zwischen 10° bis 20°, vorzugsweise 15° angestellt ist. Gegenüber der aus der WO 97/49355 bekannten D- oder Nieren-Form wird mit der vorliegenden Erfindung eine um 180° gedrehte D- oder Nieren-Form erzeugt, die eine physiologischere Einströmung vom Vorhof durch die Klappein den Ventrikel erzielt.

Weiterbildungen der Erfindung sind in den Unteransprüchen aufgeführt.

So wird vorzugsweise die durch die Stützgehäuseinnenflächen definierte Strömungsrichtung gegenüber der Basisringgrundfläche um einen Winkeln von 70° bis 80°, vorzugsweise 75° geneigt. Dies bedeutet, daß die Stützgehäuse-Innenwandung, die in Strömungsrichtung gesehen leicht konisch verläuft, in Bezug auf die durch die Verbindunglinie der Pfostenspitzen und die Strömungsrichtung bzw. entsprechende parallele Ebenen hierzu gebildete Flächen jeweils um 15° geneigt wird.

Nach einer weiteren Ausgestaltung der Erfindung stehen die Längen der halben Querachsen der Basis-Grundfläche in einem Verhältnis von 1,5 bis 2,5 : 1. Insbesondere bei einem Halbachsenverhältnis von etwa 2 : 1 läßt sich eine weitgehend der natürlichen Mitral-Klappe angenäherte Form erzielen. Die gemeinsame Längsachse der beiden unterschiedlichen Halbellipsen des Stützgehäuses besitzen eine Länge zwischen 10 mm und 45 mm. Vorzugsweise sind die Pfosten dickengleich in die Wandungen integriert, d.h. daß die Wandung im Bereich der vorerwähnten Pfosten nach oben hin ausläuft, insbesondere zu einem zumindest im wesentlichen spitzen, stirnseitigen Pfostenende, dessen Spitze jedoch vorzugsweise abgerundet ist. Um die Klappensegel im Kommissurenbereich nicht zu stark zu beanspruchen, wird nach einer weiteren Ausgestaltung der Erfindung die Verbindungslinie der Segel derart gelegt, daß sie mit der oberen Innenkante der Wandungen jeweils in einer Ebene liegt.

Der besondere Vorteil der Mitral-Herzklappe, insbesondere auch gegenüber solchen Bioprothesen als Mitralklappenersatz, bei dem erfahrungsgemäß in 50 % aller Fälle eine gerinnungshemmende Medikamentation der Patienten erforderlich ist, kann der Patient, dem eine erfindungsgemäße Mitralklappenprothese eingesetzt worden ist, auf eine Medikamenteneinnahme verzichten, da durch die neue Konstruktion eine mechanische Blutschädigung weitgehend verhindert ist.

Im Rahmen der vorliegenden Erfindung liegen auch solche Ausführungsformen, bei denen an einem aus Kunststoff gefertigten Stützgehäuse Segel aus biologischem natürlichen Material befestigt sind.

Ein Ausführungsbeispiel der Erfindung ist in den Zeichnungen dargestellt. Es zeigen:

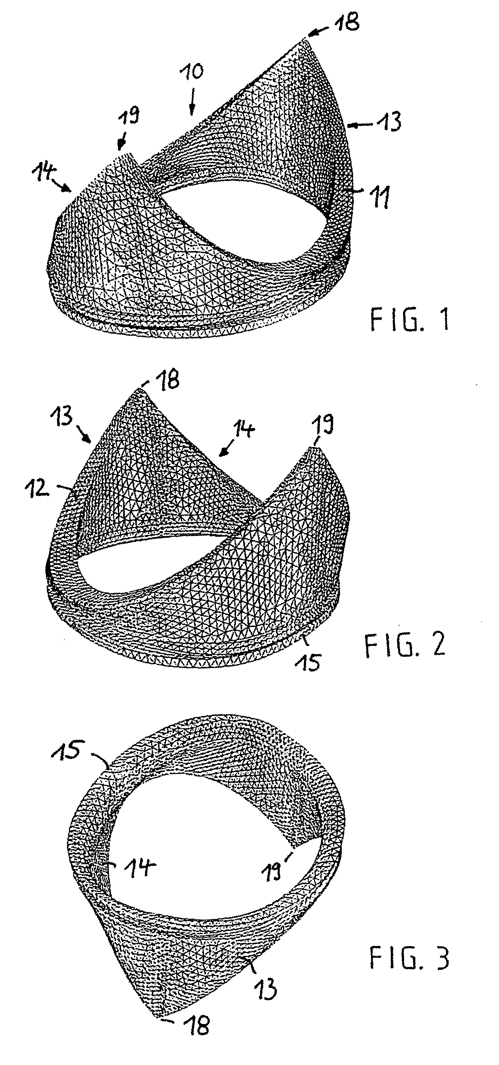

- Fig. 1 bis 3: jeweils perspektivische Ansichten der erfindungsgemäßen Mitral-Herzklappe unter verschiedenen Blickwinkeln
- Fig. 4 und 5: jeweils Draufsichten auf die Mitral-Herzklappe aus zwei Ansichten (von oben und unten) und

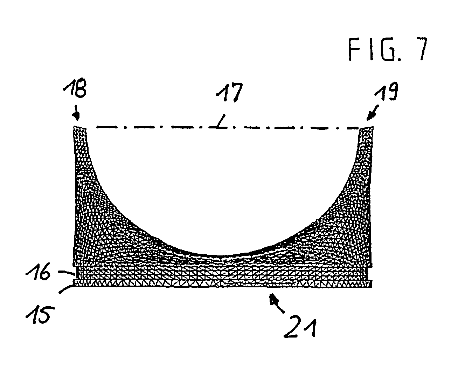

- Fig. 6 und 7: zwei Seitenansichten der Mitral-Herzklappe

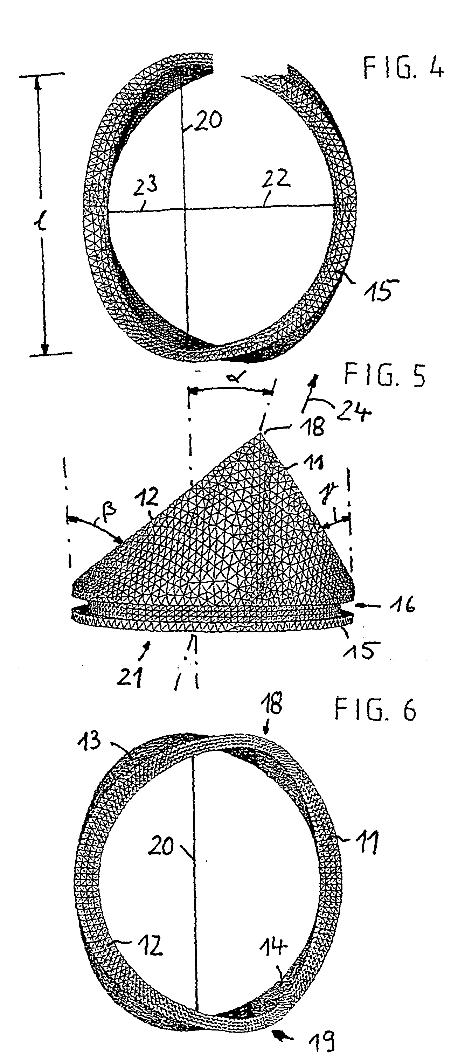

Die prothetische Mitral-Herzklappe besteht aus einem Stützgehäuse 10 mit zwei (nicht dargestellten) Segeln aus Kunststoff, vorzugsweise Polyurethan oder aus einem natürlichen Material, die auf den Stirnflächen 11, 12 der die Pfosten integrierenden Wandungen 13, 14 befestigt ist. Das Stützgehäuse 10 besteht aus einem Thermoplast, vorzugsweise aus Polyurethan, das zu einem gering biegeelastischen Körper, beispielsweise durch Spritzgießen hergestellt worden ist. Das Stützgehäuse ist einstückig und besitzt einen Basisring 15, dessen Innenkanten nach außen hin in nach dem Stand der Technik bekannter Weise abgerundet sind. Zur besseren Befestigung des Nahtrings besitzt der Basisring eine nutförmige Ausnehmung 16. Im Gegensatz zu der in der WO 97/49355 dargestellten Mitral-Herzklappe sind jedoch die Wandungen 13, 14 nicht einheitlich senkrecht zur Basisring-Grundfläche ausgerichtet, sondern um einen Winkel α von ca. 15° geneigt (siehe insbesondere Fig. 6). Eine solche Neigung ist dadurch gekennzeichnet, daß die Ebene, in der die Verbindungslinie 17 der Pfostenspitzen 18 und 19 und die gemeinsame Längsachse 20 der Pfostenbasis-Ringform liegen gegenüber der durch die gemeinsame Längsachse führende, vertikal zu der durch den Basisring gebildeten Fläche 21 liegende Ebene um 15° geneigt ist. Die Wandung 11 mit der größeren Krümmung trägt das unter einem zu der durch den Basisring 15 gebildeten Fläche stärker geneigten Winkeln angeordnete flächenkleinere (murale) Segel als die Wandung 12 mit der kleineren Krümmung. Gegenüber der aus der WO 97/49355 bekannten Form ergibt sich hierbei in einer Draufsicht nach Fig. 4 ein spiegelverkehrte D-Form, bei der die längere Halbachse 22 in einer Draufsicht auf den Basisring auf der rechten und die kürzere Halbachse 23 auf der linken Seite liegt. Durch die geometrische "Kippung" der durch die durch die Linien 17 und 20 gebildeten Ebene gegenüber der Vertikalen ergibt sich eine durch die Stützgehäuseinnenfläche definierte Strömungsrichtung 24, die gegenüber der Basisringgrundfläche um 75° geneigt ist. Das Verhältnis der Längen der halben Querachsen 22, 23 liegt im Bereich von 1,5 bis 2,5 : 1. Die gemeinsame Längsachse 20 hat eine Länge zwischen 10 mm und 45 mm. Im vorliegenden Ausführungsbeispiel sind die Pfosten dickengleich in die Wandungen 11 und 12 integriert, wobei die stirnseitigen Pfostenenden 18, 19 zumindest im wesentlichen spitz auslaufen. Die Verbindungslinie der Segel mit der oberen Innenkante 11, 12 der Wandungen 13, 14 liegen jeweils in einer Ebene. Wie aus Fig. 5 ersichtlich, ist das weniger geneigte aortale Segel etwa um β = 40° und das stärker geneigte murale Segel etwa um γ = 55° geneigt.

Die eine Draufsicht entgegen der Strömungsrichtung darstellende Fig. 6 zeigt, daß die Projektion auf die Segelauflageflächen 11 und 12 eine ebenfalls nieren- oder D-förmige Kontur besitzt, die jedoch gegenüber der durch die Basisringfläche gebildeten Kontur wiederum spiegelsymmetrisch ausgebildet ist, d.h. daß die kürzere Halbachse hier rechts angeordnet ist, also im stromabseitigen Bereich die Ausgestaltung der Segelauflageflächen in der aus der WO 97/49355 beschriebenen und dargestellten Form erhalten bleibt.

## Patentansprüche

1. Prothetische Mitral-Herzklappe, bestehend aus einem Stützgehäuse (10) mit einem Basisring (15), der zwei im wesentlichen in Ringachsrichtung weisende Pfosten trägt, die über bogenförmige, der Befestigung zweier flexibler Segel dienende Wandungen (12, 13; 11, 14) verbunden sind, wobei die freien Enden der Pfosten eine Innenauflage für die Segel bilden, wobei der Basisring - in Draufsicht betrachtet - eine geschlossene unrunde Form mit einer gemeinsamen Längsachse und zwei ungleich großen halben Querachsen (22, 23) aufweist, wobei die Pfosten auf den Enden der Längsachse (20) liegen und die Übergangsstelle von der einen zu der anderen Halbform bilden,
**dadurch gekennzeichnet,**
**daß** die Ebene, in der die Verbindungslinie (17) der Pfostenspitzen (18, 19) und die gemeinsame Längsachse (20) der Pfostenbasisring-Form liegen, gegenüber der durch die gemeinsame Längsachse (20) führenden und vertikal zu der durch den Basisring gebildeten Fläche (21) liegenden Ebene um einen Winkel (α) von 10° bis 20°, vorzugsweise 15°, geneigt ist und daß die Wandung (11 ,14) mit der größeren Krümmung das unter einem zu der durch den Basisring gebildeten Fläche stärker geneigten Winkeln angeordnete flächenkleinere, murale Segel trägt, als die Wandung (12, 13) mit der kleineren Krümmung.

2. Mitral-Herzklappe nach Anspruch 1, **dadurch gekennzeichnet, daß** die durch die Stützgehäuseinnenflächen definierte Strömungsrichtung (24) gegenüber der Basisringgrundfläche um einen Winkel von 70° bis 80°, vorzugsweise 75° geneigt ist.

3. Mitral-Herzklappe nach Ansprüche 1 oder 2, **dadurch gekennzeichnet, daß** die Segelneigung, die durch die Lage der Verbindungslinie des Segels mit der oberen Innenkante der Wandung bestimmt ist, zwischen 25° und 45° für das weniger geneigte aortale Segel und zwischen 55° und 70°, vorzugsweise 65° für das stärker geneigte murale Segel, jeweils relativ zur Basisgrundfläche, beträgt und gleichzeitig das stärker geneigte Segel um mindestens 10, vorzugsweise 20°, stärker geneigt ist als das weniger geneigte Segel.

4. Mitral-Herzklappe nach einem der Ansprüche 1 bis 3, **dadurch gekennzeichnet, daß** die Längen der halben Querachsen (22, 23) in einem Verhältnis von 1,5 bis 2,5 : 1 stehen.

5. Mitral-Herzklappe nach einem der Ansprüche 1 bis 4, **dadurch gekennzeichnet, daß** die gemeinsame Längsachse (20) eine Länge (1) zwischen 10 mm und 45 mm aufweist.

6. Mitral-Herzklappe nach einem der Ansprüche 1 bis 5, **dadurch gekennzeichnet, daß** die Pfosten dickengleich in die Wandungen integriert sind.

7. Mitral-Herzklappe nach Anspruch 6, **dadurch gekennzeichnet, daß** das stirnseitige Pfostenende (18, 19) zumindest im wesentlichen spitz ausläuft.

8. Mitral-Herzklappe nach einem der Ansprüche 1 bis 7, **dadurch gekennzeichnet, daß** die Verbindungslinie der Segel mit der oberen Innenkante (11, 12) der Wandungen (13, 14) jeweils in einer Ebene liegt.

## Claims

1. Prosthetic heart valve comprised of a support housing (10) with a base ring (15) that carries two posts extending essentially axially of the ring, connected by arcuate walls (12, 13, 11, 14) serving for the mounting of two flexible leaflets, and having outer edges forming an inside support for the respective leaflets, the base ring seen from above having a closed noncircular shape with a mutual longitudinal axis and two unequal transverse half axes (22, 23), the posts extending from the ends of the longitudinal axis (20) and forming transitions from the one to the other half
**characterized in that**
the plane including a connecting line (17) joining post tips (18, 19) and the mutual longitudinal axis (20) of the annulus of the post base ring is inclined to the plane, wherein the mutual longitudinal axis (20) lies and which is arranged vertically to the plane (21) of the basis ring, at an angle (α) of 10° to 20°, preferably 15°, and that the wall (11, 14) with the greater curvature carries the mural leaflet having a smaller surface and being inclined at a stronger angle to the base-ring plane than the wall (12, 13) with the smaller curvature.

2. Mitral heart valve according to claim 1, **characterized in that** a flow direction (24) defined by the inner surfaces of the support is inclined at an angle from 70° to 80°, preferably 75°, to a base-ring plane.

3. Mitral heart valve according to claim 1 or 2, **characterized in that** the leaflet inclination defined by the position of the connecting line of the leaflet with the upper inner edge of the respective wall, lies between 25° and 45° for the less angled aortal leaflet and between 55° and 70° preferably 65°, for the more angled mural leaflet, each in relation to the base ring plane, and at the same time the more angled leaflet is angled at at least 10°, preferably 20°, more than the less angled leaflet.

4. Mitral heart valve according to one of claims 1 to 3, **characterized in that** the lengths of the half transverse axes (22, 23) form a ratio of from 1.5:1 to 2.5:1.

5. Mitral heart valve according to one of claims 1 to 4, **characterized in that** the mutual longitudinal axis (20) has a length (I) between 10 mm and 45 mm.

6. Mitral heart valve according to one of claims 1 to 5, **characterized in that** the posts are of the same thickness as the walls and are imbedded therein.

7. Mitral heart valve according to claim 6, **characterized in that** the post ends (18, 19) are essentially pointed.

8. Mitral heart valve according to one of claims 1 to 7, **characterized in that** the connecting lines of the leaflets with the upper inner edges (11, 12) of the respective walls (13, 14) each lie in a plane.

## Revendications

1. Valvule cardiaque mitrale prothétique se composant d'un boîtier de support (10) avec un anneau de base (15) qui porte deux poteaux qui montrent pour l'essentiel dans la direction de l'axe de l'anneau et sont reliés par l'intermédiaire de parois en arc (12, 13; 11, 14) qui servent à la fixation de deux voiles flexibles, les extrémités libres des poteaux formant un appui intérieur pour les voiles, ledit anneau de base - vu de dessus - présentant une forme fermée non ronde avec un axe longitudinal commun et avec deux demi-axes transversaux (22, 23) à grandeur inégale, les poteaux étant situés sur les extrémités de l'axe longitudinal (20) et formant l'endroit de transition de l'une des demi-formes à l'autre,
**caractérisée par le fait**
**que** le plan dans lequel sont situés la ligne de jonction (17) des pointes (18, 19) des poteaux ainsi que l'axe longitudinal commun (20) de la forme se composant des poteaux et de l'anneau de base est incliné d'un angle (α) compris entre 10° et 20°, de préférence de 15° par rapport au plan qui passe à travers l'axe longitudinal (20) commun et qui est situé verticalement à la surface (21) formée par l'anneau de base, et que la paroi (11, 14) avec la courbure plus importante porte le voile mural à surface plus petite disposé à un angle plus fortement incliné par rapport à la surface formée par l'anneau de base, que la paroi (12, 13) avec la courbure plus petite.

2. Valvule cardiaque mitrale selon la revendication 1, **caractérisée par le fait que** le sens d'écoulement (24) défini par les faces internes du boîtier de support est incliné d'un angle compris entre 70° et 80°, de préférence de 75°, par rapport à la surface de base de l'anneau de base.

3. Valvule cardiaque mitrale selon la revendication 1 ou 2, **caractérisée par le fait que** l'inclinaison du voile qui est déterminée par la position de la ligne de jonction du voile avec l'arête intérieure supérieure de la paroi est comprise entre 25° et 45° pour le voile aortique à inclinaison plus faible et entre 55° et 70°, de préférence 65° pour le voile mural à inclinaison plus forte, respectivement par rapport à la surface de base, et que, en même temps, le voile à inclinaison plus forte est incliné d'au moins 10, de préférence de 20° plus fortement que le voile à inclinaison plus faible.

4. Valvule cardiaque mitrale selon l'une des revendications 1 à 3, **caractérisée par le fait que** les longueurs des demi-axes transversaux (22, 23) ) sont dans un rapport allant de 1,5 : 1 à 2,5 : 1

5. Valvule cardiaque mitrale selon l'une des revendications 1 à 4, **caractérisée par le fait que** l'axe longitudinal commun (20) présente une longueur (I) comprise entre 10 mm et 45 mm.

6. Valvule cardiaque mitrale selon l'une des revendications 1 à 5, **caractérisée par le fait que** les poteaux sont intégrés à épaisseur égale dans les parois.

7. Valvule cardiaque mitrale selon la revendication 6, **caractérisée par le fait que** l'extrémité de front (18, 19) du poteau se termine pour l'essentiel en pointe.

8. Valvule cardiaque mitrale selon l'une des revendications 1 à 7, **caractérisée par le fait que** la ligne de jonction des voiles avec l'arête intérieure supérieure (11, 12) des parois (13, 14) est située respectivement dans un plan.
